# EUROPEAN PATENT APPLICATION

(11) **EP 2 522 336 A1**
(43) Date of publication of application: **14.11.2012**
(21) Application number: 10826360.9
(22) Date of filing: 29.10.2010
(51) Int. Cl.: A61K 8/64, A61K 8/11, A61K 8/25, A61K 8/29, A61Q 3/02, A61Q 19/00

(54) **SKIN COSMETIC KNEADED COMPOSITION AND METHOD FOR PRODUCING SAME, AND METHOD FOR USING SKIN COSMETIC KNEADED COMPOSITION**

(30) Priority: 02.11.2009 JP 2009266788; 02.11.2009 JP 2009266789
(71) Applicant: Fukumotogiken Corporation Co. Ltd, Kagoshima-shi, Kagoshima 891-1105 (JP)
(72) Inventor: FUKUMOTO, Shigeru, Kagoshima-shi Kagoshima 891-1105 (JP)
(74) Representative: Plougmann & Vingtoft A/S
(86) International application number: PCT/JP2010/006400
(87) International publication number: WO 2011/052224

(57) **Abstract**

Usually, The method of polishing with a file and the likes as the management method of a nail, there is a problem also in connection with the health care administration of a nail or the skin there is much polish of the same part, and the damage on the surface of a nail becomes large, or treatment of the end of a nail or a hairline hurts its skin difficultly.

Although a pumice, a file, and the likes have ground and removed the horny layer by an elbow and the heel, there is complicatedness that it cannot be used if it is not specific places which are ground too much and depended, such as a damage to the skin, a bathroom, and a washroom.

The kneaded composition for skin cosmetics which an abrasive compound and keratin hydrolyzate were blended with the viscous base material, and was kneadeded is used.

The kneaded composition for skin cosmetics is manufactured by supplying and agitating a viscous base material, an abrasive compound, and keratin hydrolyzate in churning equipment. After applying the manufactured kneaded composition for skin cosmetics to the surface of the body part of a nail, the heel, or an elbow, it rubs and uses the applied coated article for a spreading part using fingers, a palm, or tools.

Better results will be acquired if pH 2-4 of acidic solution is used together in a spreading part.

## Description

### [Field of the invention]

The present invention relates to the kneaded composition used for a skin cosmetics article, its manufacturing process, and also its method for using.

### [Background of the invention]

In the past, the treatment of a nail is taken conventionally the method of polishing with a file and other methods. However, according to these method, the hairline of a nail and polish of the side may be difficult to treat, and may hurt their epidermis of a finger. And after polish of the nail by a file or other methods needed to wipe off without left and remove polish slag, and the surface of the nail after polish needed to apply the refractory coating for nail protection and the likes. Furthermore, such as a nail polish remover has removed after spreading of ointments such as nail polish, it also has the problem of enlarging the damage on the surface of a nail. If it is getting worth, there is a case which gives a decoration to a nail, and if keep left this for a long period of time, it makes mold possible, and various problems concerned about the health care administration of a nail

On the other hand, a pumice stone as a file and the likes, had ground and removed the horny layer of an elbow and the heel. Washed the polish part of the body in this case with warm water, and there was complicatedness that these tools could not be used if it is not specific places, such as a bathroom and a washroom, in order to wipe off wash water.

By the way, contains a keratin substance, ceramics, and the likes as cosmetics articles as a nail are proposed conventionally (for example, as the Patent documents 1). Moreover, the things using the polish effectiveness of ceramics are proposed by these people (for example, as the Patent documents 2).

### [Prior art literatures]

### [Patent]

Patent1, Japanese publication number 2008-515864
Patent2: Japanese application number 2009-146919

Although, the proposal of the Patent1 applies a keratin substance, ceramics, and the likes to the skin surface in order to only give gloss. Moreover, although the proposal of the patent documents 2 uses the polish effectiveness of ceramics, the detailed polish crack made on the surfaces of a nail by polish, and it was not able to be said the gloss on the surface of the skin is necessarily enough.

It was made in view of the above mentioned subject, the surfaces, such as a nail, the heel, or an elbow, are smoothed. And an object of the present invention is to provide the kneaded composition for skin cosmetics from which the filth adhering to these removed surfaces, its manufacturing process, and its method for using.

Moreover, while the detailed crack produced by surface smoothing is automatically restored by effectiveness of a contents component, an object of the present invention is to provide the kneaded composition for skin cosmetics in which gloss is given to this surface, its manufacturing process, and its method for using.

Furthermore, after use aims the present invention at offering the kneaded composition for skin cosmetics removed easily and promptly, its production method, and its method for using for use, even if it does not perform exceptional washing and the likes.

### [Summary of the invention]

### [The method for solving a subject]

The inventor repeated examination wholeheartedly about the subject of the above mentioned conventional technology. And found that the kneaded composition which blended ceramic particles and hydrolyzed keratin ("Keratide" (registered trademark) by Oriental feathers industrial incorporated company) with the peeling agent (gel type) generally used as cosmetics is made as an experiment, and when the object was carried out to the nail and the likes, and all the above mentioned subjects find out being solved and came to complete the present invention.

Furthermore, the kneaded composition blended ceramic particles, hydrolyzed keratin(above "Keratide") and microcapsules includes acid solution of pH 2-4 or the acid kneaded composition of pH 2-4 with the peeling agent (gel type) generally used as cosmetics is made as an experiment. And when the object was carried out to the nail and the likes, all the above mentioned subjects find out being solved and came to complete the present invention.

The kneaded composition for skin cosmetics concerning the present invention is characterized by blending an abrasive compound and hydrolyzed keratin with a viscous base material, and kneaded for skin cosmetics composition.

Second characteristic of the kneaded composition for skin cosmetics concerning the present invention is characterized by blending an abrasive compound, hydrolyzed keratin and microcapsule which includes acid solution of pH 2-4 or the acid kneaded composition of pH 2-4 with a viscous base material, and kneaded as composition.

Third characteristic of the kneaded composition for skin cosmetics concerning the present invention is characterized by blending 5-85 % of the weight of viscous base materials, 0.3-30 % of the weight of abrasive compounds, 0.1-15 % of the weight of keratin hydrolyzate, and 0.5-5 % of the weight of additives to the kneaded composition.

Fourth characteristic of the kneaded composition for skin cosmetics concerning the present invention is characterized by blending 5-85 % of the weight of viscous base materials, 0.3-30 % of the weight of abrasive compounds, 0.1-15 % of the weight of keratin hydrolyzate, 0.1-10 % of the weight of microcapsule which includes acid solution of pH 2-4 or the acid kneaded composition of pH 2-4 and 3-5 % of the weight of additives to the kneaded composition.

Fifth characteristic of the kneaded composition for skin cosmetics concerning the present invention is characterized by hydrolyzed keratin in microcapsule.

Sixth characteristic of the kneaded composition for skin cosmetics concerning the present invention is characterized by hydrolyzed keratin of the keratin which uses feathers as a raw material.

Seventh characteristic of the kneaded composition for skin cosmetics concerning the present invention is characterized by abrasive compounds being ceramic particles or hollow ceramic particles.

Eighth characteristic of the kneaded composition for skin cosmetics concerning the present invention is characterized by an additive being any one or the plurality of antiseptics, perfume, or coloring matter. Thereby, the product value of the kneaded composition for skin cosmetics increases its worth.

Ninth characteristic of the kneaded composition for skin cosmetics concerning the present invention is characterized by containing titanium oxide and/or titanium apatite. By containing titanium oxide and/or titanium apatite, to fixing of stratification of the keratin form formed on the surface of a nail and the likes with the titanium oxide and/or titanium apatite made to a firm thing.

Tenth characteristic of the kneaded composition for skin cosmetics concerning the present invention is characterized by containing Calcium salts of organic acids. By containing Calcium salts of organic acids, it is difficult of the fall in water keratin formed layer on the surface of the nail and the likes, and exhibits the effect hasten the consolidation of keratin layer formation.

The method for producing the kneaded composition for skin cosmetics concerning in the present invention, while supplying and agitating a viscous base material and hydrolyzed keratin in churning equipment, throwing in an abrasive compound gradually in the viscous solution preparation step which adjusts a viscous solution, and the viscous solution in churning equipment and distributing an abrasive compound by churning, and filling process which transports with a pump the viscous kneaded composition obtained at the viscous kneaded composition adjustment process and with a container is filled up.

Second characteristic for the method for producing the kneaded composition for skin cosmetics concerning in the present invention, while supplying and agitating a viscous base material and hydrolyzed keratin in churning equipment, throwing in an abrasive compound gradually in the viscous solution preparation step which adjusts a viscous solution, and throw in gradually the microcapsule which includes acid solution of pH 2-4, or the acid kneaded composition of pH 2-4, and a microcapsule is distributed by churning, and filling process which transports with a pump the viscous kneaded composition obtained at the viscous kneaded composition adjustment process and with a container is filled up.

The method for using the kneaded composition for skin cosmetics concerning the present invention is that after applying to the surface of the body part of either a nail, the heel, a knee or an elbow using the kneaded composition for skin cosmetics containing a viscous base material, an abrasive compound, and keratin hydrolyzate, these are characterized main by rubbing the applied kneaded composition with these spreading part using fingers, a palm, or tools.

Above mentioned abrasive compound is made by this to demonstrate the effectiveness which grinds the spreading part surface. The effectiveness for a keratin layer formed in the surface is made to demonstrate, permeating the surface of a spreading part at the above mentioned keratin hydrolyzate. The kneaded composition further applied to the viscous materials in the above mentioned viscous base material solidifies gradually, turns into many lumps, and that lumps make the effectiveness becomes easy to break away from a spreading part demonstrate.
And the detailed polish crack of the spreading part surface produced in the above mentioned polish effectiveness while the filth of the spreading part surface was smoothed by synergism, and adhered to the spreading part surface was removed the keratin layer formed above mentioned.
Moreover, a keratin layer gives gloss to the spreading part surface, and after the above mentioned polish effectiveness and a keratin layer formation effectiveness are demonstrated, these are made to secede from the kneaded composition for skin cosmetics applied above mentioned by the effectiveness of the above mentioned viscous materials further easily and more promptly than a spreading part.

Second characteristic of the method for using the kneaded composition for skin cosmetics concerning the present invention is that the above mentioned kneaded composition for skin cosmetics containing the microcapsule includes a viscous base material, an abrasive compound, hydrolyzed keratin and pH 2-4 of acid solution, or the acid kneaded composition of pH 2-4. After applying to the surface of the body part of either a nail, the heel, a knee or an elbow, it is characterized by rubbing the applied kneaded composition against this spreading part using fingers, a palm, or tools.

An abrasive compound is made to demonstrate the effectiveness which grinds the spreading part surface. Make the effectiveness forms a keratin layer in the surface demonstrate, permeating hydrolyzed keratin at the surface of a spreading part, and to a microcapsule. The effectiveness is mixed in the coated article in the acid solution or the acid kneaded composition which the envelope of the microcapsule was destroyed by the pressure to rub and suited the inside was applied, and promotes a keratin layer formation effectiveness is made to demonstrate.
The kneaded composition furthermore applied to the viscous materials in a viscous base material solidifies gradually, turns into two or more lumps, and two or more lumps make the effectiveness it becomes easy to break away from a spreading part demonstrate. While the filth which the spreading part surface was smoothed by the synergism of a more than, and adhered to the spreading part surface is removed.
While the detailed polish crack of the spreading part surface produced in the polish effectiveness is restored by the formed keratin layer. A keratin layer gives gloss to the spreading part surface, and after a polish effectiveness and a keratin layer formation effectiveness are demonstrated and made to secede from the kneaded composition for skin cosmetics applied by the effectiveness of viscous materials further easily and more promptly than a spreading part.

Third characteristic of the method for using the kneaded composition for skin cosmetics concerning the present invention is that after applying the above mentioned kneaded composition for skin cosmetics to the surface of the body part of either a nail, the heel, a knee or an elbow, as pH 2-4 of acid solution are dropped at a spreading part, or you make it mix in the kneaded composition applied the acid kneaded composition of pH 2-4, and was applied above mentioned, and it is characterized by rubbing a kneaded composition against this spreading part using fingers, a palm, or tools.

Thereby more desirable results, obtain namely. It will set by the time a coated article is made to break away from a spreading part and a spreading part dries after applying the kneaded composition for skin cosmetics to the surface of the body part of a nail, the heel, a knee or an elbow.
The above mentioned keratin layer formation effectiveness is promoted by dropping pH 2-4 of acid solution at a spreading part, or making it mix in the coated article which applied the acid kneaded composition of pH 2-4, and was applied, and rubbing a coated article against this spreading part using fingers, a palm, or tools.

Fourth characteristic of the method for using the kneaded composition for skin cosmetics concerning the present invention is that after rubbing a kneaded composition with a spreading part using fingers, a palm, or tools, and finishing agent containing water, hydrolyzed keratin, and titanium oxide.

By applying to a spreading part the finishing agent containing hydrolyzed keratin and titanium oxide, the protection layer of the keratinization layer formed in the spreading part with the kneaded composition is formed, and the fixing can be strengthened further.

Fifth characteristic of the method for using the kneaded composition for skin cosmetics concerning the present invention is that the kneaded composition for skin cosmetics which contains a viscous base material and an abrasive compound, and does not contain keratin hydrolyzate, after applying to the surface of the body part of either a nail, the heel, a knee or an elbow, the applied kneaded composition is rubbed against a spreading part using fingers, a palm, or tools, and the finishing agent which comes to blend hydrolyzed keratin and titanium oxide with water.

By applying to a spreading part the finishing agent containing hydrolyzed keratin and titanium oxide, the protection layer of the keratinization layer formed in the spreading part with the kneaded composition is formed, and the fixing can be strengthened further.

Sixth characteristic of the method for using the kneaded composition for skin cosmetics concerning the present invention is that the microcapsule includes a viscous base material, an abrasive compound and pH 2-4 of acid solution, or the acid kneaded composition of pH 2-4 is included. The kneaded composition applied after applying to the surface of the body part of either a nail, the heel, a knee or an elbow the kneaded composition for skin cosmetics which does not contain hydrolyzed keratin rubbed against a spreading part using fingers, a palm, or tools. The finishing agent comes to blend hydrolyzed keratin and titanium oxide with water.

By applying to a spreading part the finishing agent containing hydrolyzed keratin and titanium oxide, the protection layer of the keratinization layer formed in the spreading part with the kneaded composition is formed, and the fixing can be strengthened further.

The finishing agent for skin cosmetics concerning the present invention is characterized by coming to blend hydrolyzed keratin and titanium oxide with water. The protection layer of the keratinization layer formed in the spreading part with the kneaded composition is formed, and the fixing can be strengthened further.

When the cosmetics article of the conventional nail, the heel, a knee, or an elbow was used, in order for there to be a problem that is accompanied by damage not only to an application skin part but the skin of the circumference of it and to wash the skin part after application. There was complicatedness that it could not be used if it is not specific places, such as a washroom. If the kneaded composition for skin cosmetics of the present invention is used, there will be no damage to the skin in the circumference of an application skin part, and the outstanding effect of being easily anywhere applicable will be generated.
Moreover, washing after application is unnecessary, it remains as it is, a soil is removed finely, a nail turns into a glossy nail, and a cosmetics article of a nail, the heel, a knee, and an elbow with which the heel, a knee, and an elbow serve as a smooth surface is offered.

### [Effectiveness of the invention]

Moreover, according to the method of making the kneaded composition for skin cosmetics concerning the present invention generate the outstanding effect that the kneaded composition which is excellent as a cosmetics article of a nail, the heel, a knee, and an elbow can be obtained easily.

By the method for producing the kneaded composition for skin cosmetics concerning in the present invention, it will be granted the kneaded composition applied to a nail for skin cosmetics concerning the present invention, and it applies to the heel, a knee, and an elbow, the outstanding effect that they serve as advanced beauty.

By the method for using the kneaded composition for skin cosmetics concerning in the present invention, it will be granted a glossy nail if the kneaded composition applied to a nail according to the method for using for the kneaded composition for skin cosmetics concerning the present invention, and it applies to the heel, a knee, and an elbow, the outstanding effect that they serve as a smooth surface.

### [Explanation for Figures]

[Fig. 1] Perspective diagram showing the nail surface, before applying the kneaded composition for skin cosmetics concerning the present invention.
[Fig. 2] Perspective diagram showing the nail surface, after applying the kneaded composition for skin cosmetics concerning the present invention.

### [The prefer execution for the invention]

In the first execution of the present invention, the kneaded composition for skin cosmetics concerning to add and kneaded an abrasive compound and hydrolyzed keratin to the viscous base material containing the viscous materials used for conventional cosmetics and drugs, and it is desirable preferably to add a proper quantity of solvents and/or water.
In addition, the combination presentation of the kneaded composition for skin cosmetics in the present invention is not limited to the composition of this execution form.

Generally as a viscous base material, the gel like peeling agent (viscous materials are contained) used can also be used as cosmetics. As a peeling agent, the others and the organic powder and the end of inorganic powder which are alpha-hydroxy acid, such as glycolic acid and lactic acid, and the likes can be used or an aqueous gel formation macromolecule can be used.
Especially as viscous materials contained in a viscous base material, it is not limited and the adhesives component used for a conventional paste and solid paste can be used.
For example, polyvinyl pyrrolidone of the average molecular weight 10,000-3 million, polyvinylalcohol, synthetic resins, such as acrylic ester, methacrylic acid ester, polyvinyl acetate, and polyvinyl butyral, natural resins, such as cellulose, such as methyl cellulose and ethyl cellulose, a starch, gelatin, and cornstarch, and the likes are mentioned. And these can be used by two or more sorts independent or mixed. The content of a viscous base material is 5-50 % of the weight to the kneaded composition.

Especially as an abrasive compound, it is not limited and various abrasive compounds can be used.
For example, ceramic particles, milt origin particles, glass particles, coral particles, saw dust particles, rubber particles, and the likes are mentioned. It is desirable when using the destroyed particles, and few ceramic particles or hollow ceramic particles of an impurity stops the damage to the skin surface to the minimum. These one sort or two more sorts can be used or mixed. The content of an abrasive compound is 0.3 to 30 % of the weight preferably 15 to 25 % of the weight to the kneaded composition.

Especially as keratin hydrolyzate, not limited and keratin originating in various animals can be used. And these one sort or two more sorts can be used or mixed.
The content of hydrolyzed keratinis 0.1 to 15 % of the weight preferably 1 to 3 % of the weight to the composition.

Antiseptics, perfume, coloring matter, and the likes can be used as an additive blended with a viscous base material. These one sort or two more sorts can be used or mixed.
As for perfume or coloring matter, what is used for conventional cosmetics, drugs, and the likes are desirable.
The content of an additive is 0.1 to 5 % of the weight preferably 3 to 5 % of the weight to the composition.

The kneaded composition for skin cosmetics can be manufactured by throwing in and agitating a viscous base material, an abrasive compound and keratin hydrolyzate, and an additive in churning equipment.

Namely, compositions becomes impossible to dissolve and view as solid components (hydrolyzed keratin and a viscous base material are included) other than an abrasive compound from churning, and churning distributes and they change
an abrasive compound into the state where a massive is no longer viewed.
It agitates more preferably, heating the composition except an abrasive compound among the kneaded compositions for skin cosmetics if needed with churning equipment,
With the viscous solution preparation process changed into the state where it becomes impossible to dissolve and view, the composition of the solid of the compositions except an abrasive compound ranks second, the viscous kneaded composition preparation process changed into the state where the abrasive compound of predetermined quantity is gradually fed into a viscous solution, an abrasive compound distributes in a viscous solution, and a massive thing is no longer viewed agitating while continuing heating if needed. It can manufacture with the production method which consists of each process of the viscous kneaded composition filling process which transports a viscous kneaded composition with a pump, and with a product container is filled up.

Furthermore, in a viscous kneaded composition adjustment process, titanium oxide can also be added. Titanium oxide can be added 0.05 to 1 % of the weight to the composition.

The kneaded composition for skin cosmetics of the present invention is used as follows.

Namely, by rubbing the applied coated article against a spreading part using fingers, a palm, or tools, after applying to the surface of the body part of a nail, the heel, or an elbow, make an abrasive compound demonstrate the effectiveness which grinds the spreading part surface, and the effectiveness which forms a keratin layer in the surface is made to demonstrate, permeating hydrolyzed keratin at the surface of a spreading part, the kneaded composition further applied to the viscous materials in a viscous base material solidifies gradually, and it turns into some lumps and two or more lumps make the effectiveness, it becomes easy to break away from a spreading part demonstrate.
And the spreading part surface is smoothed by synergism more than, and the filth adhering to the spreading part surface is removed.
Moreover, while the detailed polish crack of the spreading part surface produced in the polish effectiveness is restored by the keratin layer formed.
A keratin layer gives gloss to the spreading part surface, and after a polish effectiveness and a keratin layer formation effectiveness are demonstrated, you are made to secede from the kneaded composition for skin cosmetics applied by the effectiveness of viscous materials further easily and more promptly than a spreading part.

More desirable results are acquired by using the kneaded composition for skin cosmetics of the present invention as follows.

Namely, by the time a coated article is made to break away from a spreading part in the above mentioned direction for use after applying the kneaded composition for skin cosmetics to the surface of the body part of a nail, the heel, or an elbow and a spreading part dries, it will set. The above mentioned keratin layer formation effectiveness is promoted by dropping pH 2-4 of acid solution at a spreading part, or making it mix in the coated article which applied the acid kneaded composition of pH 2-4, and was applied, rubbing a coated article against a spreading part using fingers, a palm, or tools, and applying the same direction for use as the above below.

The pH 2-4 acid solution in particular are not limited, and what was diluted until it presented pH 2-4 as an organic acid solution or an inorganic acid aqueous solution can be used for them.
Otherwise, a cation can be given to the skin surface, or the cosmetics component aiming at moisturizing or the cosmetics oil component for gloss promotion can be blended, or a microcapsule can be made to be able to include these in these acid solution, and they can also be blended with them.

In the further above mentioned direction for use, after rubbing a kneaded composition against a spreading part using fingers, a palm, or tools, the finishing agent containing water, hydrolyzed keratin, and titanium oxide can also be applied to a spreading part.
By applying the finishing agent to a spreading part, the protection layer of the keratinization layer formed in the spreading part with the kneaded composition is formed, and the fixing can be strengthened further.

The kneaded composition for skin cosmetics applied to the present invention in the 2^{nd} execution form of the present invention to the viscous base material containing the viscous materials used for, and conventional cosmetics and drugs.
The microcapsule which includes an abrasive compound, hydrolyzed keratin and pH acid solution, or the acid kneaded composition of pH 2-4 is added and kneaded, and it is desirable to add a proper quantity of desirable solvents and/or water.
In addition, the combination presentation of the kneaded composition for skin cosmetics in the present invention is not limited to the composition of this execution form.

Use the composition described with the 1^{st} upper execution form about each of a viscous base material, an abrasive compound, and hydrolyzed keratin. A microcapsule include pH 2-4 of acid solution, or includes the acid kneaded composition of pH 2-4 is included 0.1 to 10 % of the weight to the composition, preferably 1 to 5 % of the weight to the composition.

As a microcapsule , for example, a microcapsule which include pH 2-4 of acid solution, or includes the acid kneaded composition of pH 2-4, what was prepared by which well known method of the mechanical techniques, such as the physicochemical techniques, such as chemical methods, such as an interfacial-polymerization method and the in-site polymerizing method, a dry technique in liquid, the gel converting method, and the coacervation method, spray drying process, and a dry-blending method, can be used.
Since a microcapsule includes pH 2-4 of acid solution which are aqueous solutions or includes the acid kneaded composition of pH 2-4, it can make hydrophobic oil able to carry out W/0 emulsification of acid solution or the acid kneaded composition, and can be prepared by the method of using this emulsified matter as an intension thing.
Furthermore, the hydrophobic oil instead of a water medium can be made to be able to distribute acid solution or an acid kneaded composition, and it can also use for preparation of a microcapsule.

The pH 2-4 of acid solution or an acid kneaded composition, was diluted until it presented pH 2-4 as not the thing limited especially but an organic acid solution or an acid kneaded composition, or an inorganic acid aqueous solution or an acid kneaded composition can be used, and an acetic acid solution or an acetic acid kneaded composition is used preferably.

A microcapsule can be made to be able to include either pH 2-4 of acid solution, or the acid kneaded composition of pH 2-4, or another microcapsule can be made to include acid solution and an acid kneaded composition so that each may separate.
Moreover, an aiming at moisturizing cosmetics giving a cation to these acid solution or an acid kneaded composition on the skin surface otherwise component or glaze, the cosmetics oil component for and the likes can also be blended.
Moreover, these additional components can also be used for the kneaded composition for skin cosmetics, blending them together with the microcapsule which makes the microcapsule of another kind include and includes above mentioned acid solution or acid kneaded composition of the same kind.

The film material of a microcapsule can use either of a well known material, for example, gelatin, gum arabic, urea resin, urethane resin, an acrylate resin, a melamine resin, and polyamide resin are mentioned.

A well known dispersant can be used in preparation of a microcapsule. As a dispersant, for example Alkyl sulfuric acid ester salt, an alkyl sulfonic acid salt, anion nature surface active agents, such as alkylbenzene sulfonates, an alkyl naphthalenesulfonic acid salt, or dialkylsulfosuccinate; polyoxyethylene alkyl ether, non-ion system surface active agents, such as polyoxyethylene alkyl aryl ether or polyoxyethylene fatty acid ester; partial saponification polyvinyl alcohol, low-grade alkyl ether denaturation polyvinyl alcohol, an acrylamide polymer, water soluble polymer compounds, such as an acrylamide copolymer, polyacrylate, a polystyrene sulfonate salt, maleic anhydride and an isobutylene copolymer salt, carboxymethylcellulose salt, hydroxyethyl cellulose, starch, casein, gum arabic, or gelatin, are mentioned.

Although the particle diameter of the microcapsule is united with the purpose and can be set also to a gap, the range of it is 1 to 50 micrometers more preferably 0.5 to 500 micrometers.

In the 2nd execution form, the above mentioned kneaded composition for skin cosmetics can be manufactured by throwing in and agitating the microcapsule and additive which include a viscous base material, an abrasive compound, hydrolyzed keratin, pH 2-4 of acid solution, or the acid kneaded composition of pH 2-4 in churning equipment.

Namely, among compositions, it becomes impossible to dissolve and view solid components (hydrolyzed keratin and a viscous base material are included) other than an abrasive compound from churning, and churning distributes and they change an abrasive compound and a microcapsule into the state where a massive thing is no longer viewed. It is a group excluding the abrasive compound among the kneaded compositions for skin cosmetics more preferably, agitate heating compounds if needed with churning equipment, and the composition of the solid (keratin hydrolyzate and a viscous material are included) of the compositions except an abrasive compound and a microcapsule ranks second with the viscous solution preparation process which will be in the state where it becomes impossible to dissolve and view, the abrasive compound of predetermined quantity is gradually fed into a viscous solution, agitating, while continuing heating if needed. Furthermore, the microcapsule which includes pH 2-4 of acid solution of predetermined quantity or the acid kneaded composition of pH 2-4 is gradually fed into a viscous solution, an abrasive compound and a microcapsule distribute in a viscous solution, and it can manufacture with the production method which consists of each process of the viscous kneaded composition preparation process which will be in the state where a massive thing is no longer viewed, and the viscous kneaded composition filling process which rank second and transport a viscous kneaded composition with a pump, and with which a product container is filled up.

Furthermore in a viscous kneaded composition adjustment process, titanium oxide can also be added titanium oxide is made into 0.05 to 1 % of the weight to the composition.

The kneaded composition for skin cosmetics of the present invention is used as follows.

Namely, by rubbing the applied coated article against a spreading part using fingers, a palm, or tools, after applying to the surface of the body part of a nail, the heel, or an elbow, make an abrasive compound demonstrate the effectiveness which grinds the spreading part surface, make the effectiveness which forms a keratin layer in the surface demonstrate, permeating hydrolyzed keratin at the surface of a spreading part, and to a microcapsule.
The effectiveness which is mixed in the coated article in which the acid solution or the acid kneaded composition which the envelope of the microcapsule was destroyed by the pressure to rub and suited the inside was applied, and promotes a keratin layer formation effectiveness is made to demonstrate.
The kneaded composition furthermore applied to the viscous materials in a viscous base material solidifies gradually, turns into two or more lumps, and two or more lumps make the effectiveness it becomes easy to break away from a spreading part demonstrate.
And the spreading part surface is smoothed by synergism, and the filth adhering to the spreading part surface is removed.
Moreover, while the detailed polish crack of the spreading part surface produced in the polish effectiveness is restored by the keratin layer formed. A keratin layer gives gloss to the spreading part surface, and after a polish effectiveness and a keratin layer formation effectiveness are demonstrated, you are made to secede from the kneaded composition for skin cosmetics applied by the effectiveness of viscous materials further easily and more promptly than a spreading part.

More desirable results are acquired by using the kneaded composition for skin cosmetics of the present invention as follows.

Namely, by the time a coated article is made to break away from a spreading part in the above mentioned direction for use after applying the kneaded composition for skin cosmetics to the surface of the body part of a nail, the heel, or an elbow and a spreading part dries, it will set, The above mentioned keratin layer formation effectiveness is promoted by dropping pH 2-4 of acid solution at a spreading part, or making it mix in the coated article which applied the acid kneaded composition of pH 2-4, and was applied, rubbing a coated article against a spreading part using fingers, a palm, or tools, and applying the same direction for use as the above below.

In the further above mentioned direction for use, after rubbing a kneaded composition against a spreading part using fingers, a palm, or tools, the finishing agent containing water, hydrolyzed keratin, and titanium oxide can also be applied to a spreading part.
By applying the finishing agent to a spreading part, the protection layer of the keratinization layer formed in the spreading part with the kneaded composition is formed, and the fixing can be strengthened further.

In the 3rd execution form of the present invention, kneaded the kneaded composition for skin cosmetics concerning the present invention without
including the viscous base material and abrasive compound containing the viscous materials used for conventional cosmetics and drugs and including hydrolyzed keratin.
And as direction for use of this kneaded composition for skin cosmetics,
After applying to the surface of the body part of either a nail, the heel, a knee or an elbow, the applied kneaded composition is rubbed against a spreading part using fingers, a palm, or tools, and the finishing agent which comes to blend keratin hydrolyzate and titanium oxide with water is applied to a spreading part after that.

By applying to a spreading part the finishing agent containing hydrolyzed keratin and titanium oxide, the protection layer of the keratinization layer formed in the spreading part with the kneaded composition is formed, and the fixing can be strengthened further.

In the 4th execution form of the present invention,
The kneaded composition for skin cosmetics concerning the present invention is also kneaded without including the microcapsule which includes the viscous base material, the abrasive compound and pH 2-4 of acid solution, or the acid kneaded composition of pH 2-4 containing the viscous materials used for conventional cosmetics and drugs and including hydrolyzed keratin.
And as direction for use of this kneaded composition for skin cosmetics, After applying to the surface of the body part of either a nail, the heel, a knee or an elbow, the applied kneaded composition is rubbed against a spreading part using fingers, a palm, or tools, and the finishing agent which comes to blend keratin hydrolyzate and titanium oxide with water is applied to a spreading part after that.

By applying to a spreading part the finishing agent containing hydrolyzed keratin and titanium oxide, the protection layer of the keratinization layer formed in the spreading part with the kneaded composition is formed, and the fixing can be strengthened further.

For the kneaded composition can use titanium apatite instead of titanium oxide, or can use mix of these.

Although constituted as mentioned above, the kneaded composition for skin cosmetics of the present invention is not limited to the above mentioned execution form, within the limits of the technical thought of the present invention, can be changed variously and can be carried out.

### [Examples]

Next, although the present invention is explained still in detail based on a case of the operation, the present invention is not limited to the following case of the operation.
Table 1 shows the examples from 1 to 5. Table 2 shows the evaluation results for the examples 1 and 2 with the comparative examples from 1 to 3. Table 3 shows the evaluation results for the examples from 3 to 7, ⓞ is very good evaluation , O is good, Δ is neutral and × is bad.

As the Example 1, 200 g of commercial peeling gel (trade name "Kurodeitan Scrub Gel" made by B & C Raboratories) is put into an agitation vessel as a viscous base material. It is seasoned with 0.1 wt% of an apple perfume and 0.4 wt% of hydrolyzed keratin(trade name "Keratide" made by Oriental feathers industrial incorporated company) which is made from waterfowl feathers as a raw material. After agitating until the hydrolyzed keratin and the perfume dissolve completely and become transparent, 20 wt% of a powder of hollow ceramic particles (40-100 microns of average diameter) refined as an abrasive compound were added gradually, and agitated until the complete dispersion, and finished the kneaded composition for skin cosmetics.
This kneaded composition was pressure-fed with the peristaltic pump, into a commercial inner tube container with about 5 g at a time, the lid was given, and was kept at the room temperature.

In the Examples 2 and 3, each the kneaded composition contains of the 0.4 wt% of titanium oxide or titanium apatite respectively, as an adhesive agent between nail and the keratin hydrolyzate.
And in the Example 4, the composition contains of the 0.4 wt% of calcium hydroxide salt as an aggregating agent of the keratin hydrolyzate.
And as the Example 5, the composition contains of the 2.0 wt% of microcapsules, as a hardener of the aggregate of the keratin hydrolyzate.

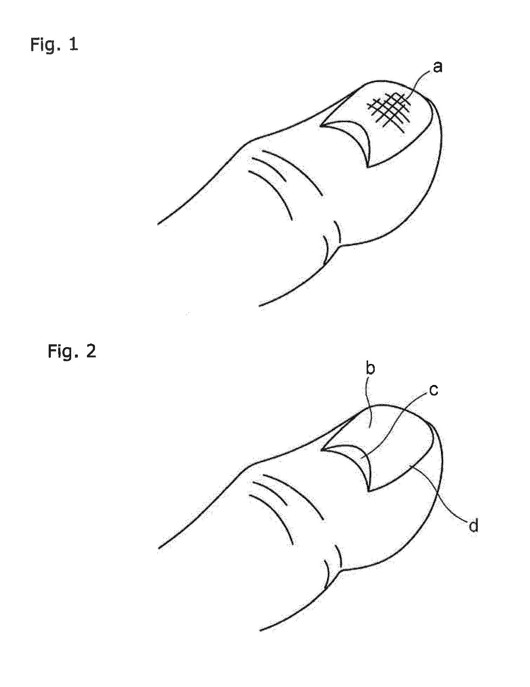

Commercial vinegar was diluted with water, and was adjusted to about pH 3.5, and acid solution put in and used it for a 10ml dropping container. Press out the kneaded composition for skin cosmetics manufactured in case of the Example 2 from an inner tube container.
The kneaded composition became some lumps and was automatically removed from the surface of the nail as moisture fell out from the kneaded composition by the
for 1 minute grade, when a small quantity was put on the surface of the nail, it extended with fingers and the spreading part was lightly rubbed with fingers.
When one drop of above mentioned acid solution were placed on the surface of the nail, were applied and were extended immediately after that, as finally shown in Fig. 2, gloss came out of it to the nail while the soil of the nail was removed.
In case of the Example 5,the above mentioned effect was gained without the use of the acid solution.

As the Example 2, the titanium oxide 1g was added and agitated to the skin cosmetics public funds kneaded composite of the case of the Example 1, and the mixed solution for skin cosmetics was obtained.

As the Example 3, 1 g of titanium apatite was added and agitated to the skin cosmetics public funds kneaded composite of the case of the Example 1, and the mixed solution for skin beauty was obtained.

As the Example 4, the calcium acetate salt 1g was added and agitated as calcium hydroxide salt to the skin cosmetics public funds kneaded composite of the case of the Example 1, and the mixed solution for skin cosmetics was obtained.

And in the Example 5, the microcapsule includes an acid aqueous solution such as a commercial vinegar diluted with water and adjusted to about pH 3.5. The microcapsules were thrown into the kneaded composition gradually,and agitated until the clusters of the microcapsule particles disappeare, and finished the mull composite for skin cosmetics.

The microcapsule particles were prepared by a professional researcher and given to us for the use. Gelatin was used as the film material, Tween80 was used as the dispersant, snd an oil was used to make the W/0 emulsion of the acid aqueous solution of the above-mentioned pH3.5, and the emulsion was made up to the microcapsule paticles by the method for the intended use.
The reason why the microcapsule technique is adopted is, for delaying the hardening effect of the acid in the using scene of the product.
The compositions which gained in the Example 5, press out the kneaded composition for skin cosmetics manufactured in case of the operation 2 from an inner tube container, put a small quantity on the surface of a nail, and it extends with fingers,
The kneaded composition became some lumps and it was automatically removed from the surface of the nail, and finally, the soil of the nail was removed and gloss came out of it to the nail as moisture fell out from the kneaded composition in about several minutes, when the spreading part was lightly rubbed with fingers.

In the example 6, the finishing solution, the composition of which: dissolving water with agitation 78.5 g; keratin hydrolyzate 20 g; titanium oxide 0.5 g; perfume 0.5 g; antiseptics 0.5 g, was prepared. The kneaded composition which gained in the Example 1, was put a small quantity on the surface of a nail from an inner tube container, and was extended with fingers. Then the finishing solution was applied to the nail.
And in the example 7, the finishing solution, the composition of which: dissolving water with agitation 70 g; keratin hydrolyzate 20 g; titanium oxide 0.5 g ; perfume 0.5 g ; antiseptics 0.5 g ; calcium acetate salt 0.5 g,
was prepared. The kneaded composition which gained in the Example 1, was put a small quantity on the surface of a nail from an inner tube container, and was extended with fingers. Then the finishing solution was applied to the nail.

### [Industrial availability]

As the Examples 2,4 and 5,they are used as the general consumers oriented cosmetics articles and cosmetics articles for contractors, such as a nail salon and an esthetic salon.
Since there is an effectiveness which abolishes the crack in which the skin surface is small, it is available also as a medical supply for a hospital nurse and nursing-care-for-elderly-people institution employees, for example. Moreover, since there is an effectiveness which the toughness and plasticity of a nail also increase when applying to a nail, string players, such as a harp and a guitar, use a pick and the likes.
Since hurting one's nail is lost even if it performs by a naked nail without things for a long time, it is available also as the nail cosmetics and a reinforcement article for music players.

### [Explanation of a code]

a : Part from the cracks of the crack ground with natural crack or file and the likes.
b : Nail surface on which the cracks were removed.
c : The part on which the cracks of the hairline of nail were removed.
d : The part on which the cracks of both ends of nail were removed.

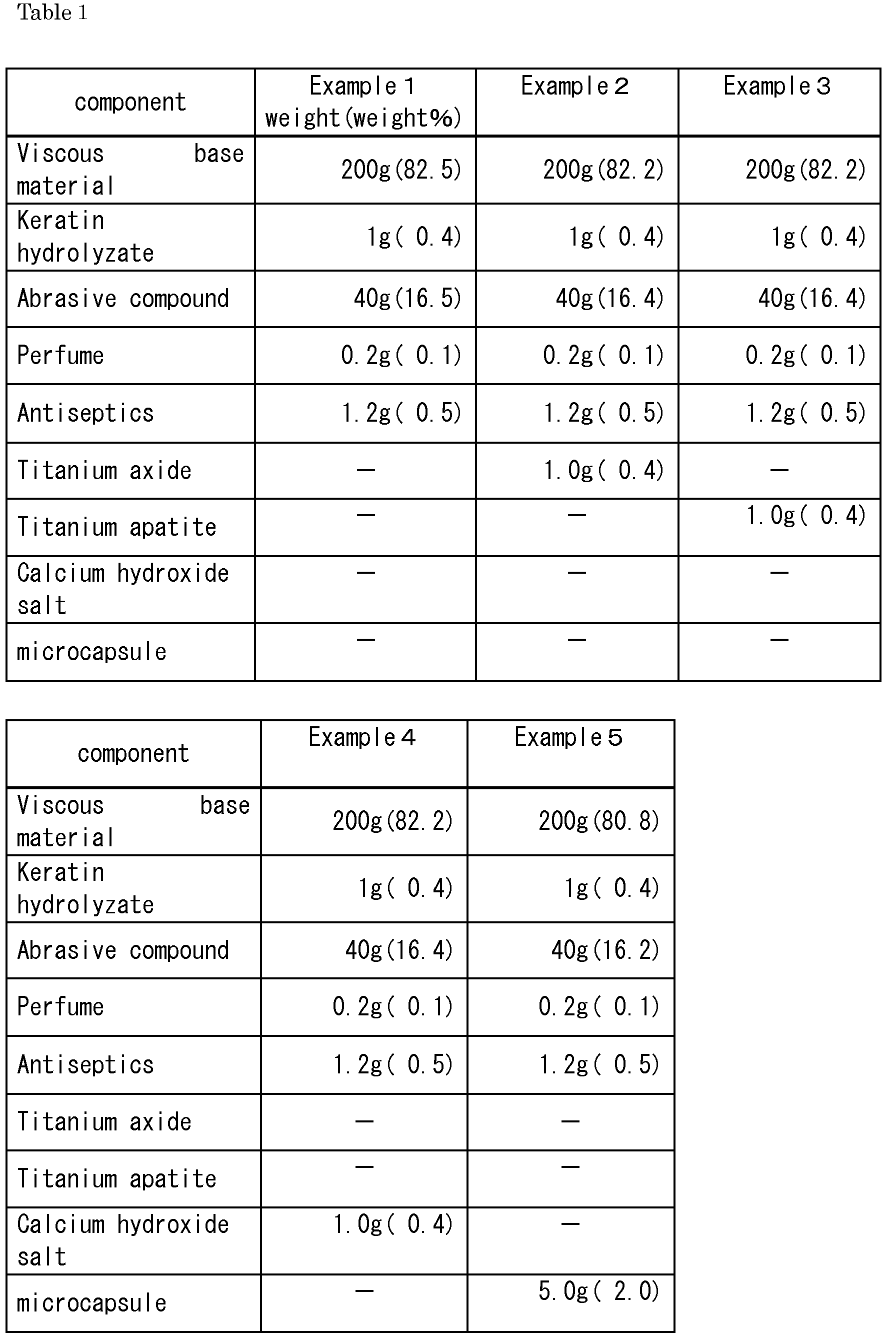

**Table 2**

| Evalution item | Comparative example 1 | Comparative example 2 | Comparative example 3 | Example 1 | Example 2 |
|---|---|---|---|---|---|
| Self-healing capabilities | × | × | × | ⓞ | ⓞ |
| Aesthetics | ⓞ | ⓞ | ⓞ | O | O |
| Sustainability | ⓞ | ⓞ | ⓞ | O | O |
| Drying | × | × | × | ⓞ | ⓞ |
| Crack | Δ | O | O | ⓞ | ⓞ |
| Scratch | ⓞ | ⓞ | ⓞ | ⓞ | ⓞ |
| Hygiene | × | Δ | Δ | ⓞ | ⓞ |
| Mold | × | Δ | Δ | ⓞ | ⓞ |
| Nail trichophyton | × | Δ | Δ | ⓞ | ⓞ |
| Respiration | × | × | × | ⓞ | ⓞ |
| Treatment time | × | Δ | Δ | O | O |
| Care after treatment | Δ | Δ | × | ⓞ | ⓞ |
| fixability | × | O | O | Δ | O |

| | | | | | |
|---|---|---|---|---|---|
| Comparative example 1 : nail extension (aceton-type), Comparative example 2 : top coat (aceton-type), Comparative example 3 : gel nail (acrylic resin-type) | | | | | |

**Table 3**

| Evalution item | Example 3 | Example 4 | Example 5 | Example 6 | Example 7 |
|---|---|---|---|---|---|
| Self-healing capabilities | ⓞ | ⓞ | ⓞ | ⓞ | ⓞ |
| Aesthetics | O | O | O | O | O |
| Sustainability | O | O | O | O | O |
| Drying | ⓞ | ⓞ | ⓞ | ⓞ | ⓞ |
| Crack | ⓞ | ⓞ | ⓞ | ⓞ | ⓞ |
| Scratch | ⓞ | ⓞ | ⓞ | ⓞ | ⓞ |
| Hygiene | ⓞ | ⓞ | ⓞ | ⓞ | ⓞ |
| Mold | ⓞ | ⓞ | ⓞ | ⓞ | ⓞ |
| Nail trichophyton | ⓞ | ⓞ | ⓞ | ⓞ | ⓞ |
| Respiration | ⓞ | ⓞ | ⓞ | ⓞ | ⓞ |
| Treatment time | O | O | O | O | O |
| Care after treatment | ⓞ | ⓞ | ⓞ | ⓞ | ⓞ |
| fixability | O | ⓞ | ⓞ | ⓞ | ⓞ |

## Claims

1. A kneaded composition for skin cosmetics comprising at least a viscous base material, a blending an abrasive compound, a keratin hydrolyzate, where these materials are kneaded to gain the composition.

2. A kneaded composition for skin cosmetics comprising an aqueous acid of pH 2-4 or a microcapsule including a composition of pH 2-4, where these materials are kneaded to gain the composition.

3. The kneaded composition for skin cosmetics of claim 1, including 5 to 85 % of the weight of the viscous base materials, 0.3 to 30 % of the weight of the abrasive compounds, 0.1 to 15 % of the weight of the keratin hydrolyzates, and 0.1 to 5 % of the weight of additives to the kneaded composition, based on the entire weight of the composition.

4. The kneaded composition for skin cosmetics of claim 2, including 0.1 to 10 % of the weight of microcapsule which includes aqueous acids of pH 2-4 or the acid kneaded composition of pH 2-4, and 0.1 to 5 % of the weight of additives to the kneaded composition, based on the entire weight of the composition.

5. The kneaded composition for skin cosmetics in claim 2, wherein keratin hydrolyzate is included by the microcapsule.

6. The Kneaded composition for skin cosmetics in claim 1 to 5, wherein the keratin hydrolyzate is the hydrolyzate of the keratin from feathers.

7. The kneaded composition for skin cosmetics in claim 1 to 6, wherein the abrasive compounds is ceramic particles or hollow ceramic particles.

8. The kneaded composition for skin cosmetics in claim 1 to 7, wherein the additive comprises of any one or a plurality of antiseptics, perfume, or coloring matter.

9. The kneaded composition for skin cosmetics in claim 1 to 8, further including titanium oxide and / or titanuimu apatite.

10. The kneaded composition for skin cosmetics in claim 1 to 9, further including an organic acid calsium salt.

11. A method of making a kneaded composition for skin cosmetics, comprising churning equipment, a viscous base material and keratin hydrolyzate are supplied and agitated, having the viscous kneaded composition adjustment process of adjusting the viscous kneaded composition which carried out, adding an abrasive compound gradually and agitating it in the viscous solution preparation step which adjusts a viscous solution, and the viscous solution in churning equipment, and in which the abrasive compound was distributed.

12. A method of making a kneaded composition for skin cosmetics comprising churning equipment, a viscous base material and keratin hydrolyzate are supplied and agitated, and while carrying out, adding an abrasive compound gradually in the viscous solution, preparation step which adjusts a viscous solution, and the viscous solution in churning equipment and distributing an abrasive compound by churning, adding gradually the microcapsule which includes pH 2-4 of aqueous acids, or the acid kneaded composition of pH 2-4, and a microcapsule is distributed by churning, the method of making the kneaded composition for skin cosmetics **characterized by** having a viscous kneaded composition preparation process which adjusts the viscous kneaded composition in which the abrasive compound and the microcapsule were distributed by this.

13. A method for using the kneaded composition for skin cosmetics in claim 1 to claim 9, comprising rubbing the applied kneaded composition against a spreading part using fingers, a palm, or tools after applying a composition to the surface of the body part of either a nail, the heel, a knee or an elbow.

14. The method for using the kneaded composition for skin cosmetics in claim 13 wherein after rubbing the applied kneaded composition against a spreading part using fingers, a palm, or tools after applying a composition to the surface of the body part of either a nail, the heel, a knee or an elbow, throw in gradually pH 2-4 of aqueous acids, or the acid kneaded composition of pH 2-4, and the viscous solution in churning equipment, and in which the abrasive compound was distributed by rubbing the applied kneaded composition against a spreading part using fingers, a palm, or tools.

15. A method for using for the kneaded composition for skin cosmetics in claim 13 or 14 **characterized by** the kneaded composition was rubbed against the spreading part using fingers, a palm, or tools applying to a spreading part the finishing agent which contains water, keratin hydrolyzate, and titanium oxide in the back.

16. A method for using for the kneaded composition for skin cosmetics **characterized by** the step which contains a viscous base material and an abrasive compound, and
does not contain keratin hydrolyzate after applying the kneaded composition for cosmetics to the surface of the body part of either a nail, the heel, a knee or an elbow, and after that there is a rubbing step which the applied kneaded composition against a spreading part using fingers, a palm, or tools, and applying to a spreading part after that the finishing agent which comes to blend keratin hydrolyzate and titanium oxide with water.

17. A method for using for the kneaded composition for skin cosmetics **characterized by** the step which contains a viscous base material, an abrasive compound, and pH 2-4 of aqueous acids, or pH 2-4 of the kneaded composition for skin cosmetics which contains the microcapsule, which includes an acid kneaded composition and does not contain keratin hydrolyzate, after applying to the surface of the body part of either a nail, the heel, a knee or an elbow, and after that there is a rubbing step which the applied kneaded composition against a spreading part using fingers, a palm, or tools, and applying to a spreading part after that the finishing agent which comes to blend keratin hydrolyzate and titanium oxide with water.

18. A finishing agent for skin cosmetics by which it is characterized of blend keratin hydrolyzate and titanium oxide with water.

19. The finishing agent for skin cosmetic in claim 18, wherein the agent is characterized of hydrated calcium salt.
